# EUROPÄISCHE PATENTANMELDUNG

(11) **EP 2 732 784 A1**
(43) Veröffentlichungstag der Anmeldung: **21.05.2014**
(21) Anmeldenummer: 13189869.4
(22) Anmeldetag: 23.10.2013
(51) Int. Cl.: A61B 18/14, A61B 18/00, A61F 2/82

(54) **Hochfrequenz-Applikationsvorrichtung zum vaskulären Einsatz, insbesondere zur Applikation von Hochfrequenz-Energie an der Renal-Arterienwand**

(30) Priorität: 20.11.2012 US 201261728251 P
(71) Anmelder: Biotronik AG, 8180 Bülach (CH)
(72) Erfinder: Bakczewitz, Frank, 18055 Rostock (DE); Degen, Nicolas, 8222 Beringen (CH); Hofmann, Eugen, 8049 Zürich (CH)
(74) Vertreter: Lindner-Vogt, Karin L.

(57) **Zusammenfassung**

Eine Hochfrequenz-Applikationsvorrichtung zum vaskulären Einsatz, insbesondere zur Applikation von Hochfrequenz(HF)-Energie an der Renal-Arterienwand, umfasst
- einen Katheter (1) mit einem ihn in Längsrichtung durchsetzenden Lumen (4),
- einen im Lumen (4) geführten, selbstexpandierenden, stentartigen Träger (6), und
- einen am Träger (6) angeordneten HF-Applikator (9) zur Abgabe von HF-Energie an Körpergewebe,
- wobei der HF-Applikator (9) als Multipol-Anordnung eine Mehrzahl von über den Träger (6) axial und peripher verteilt angeordneten HF-Kontaktelementen (14) aufweist, die vom Träger (6) isoliert sind und mit einer HF-Quelle (10) zur simultanen oder sequenziellen HF-Energie-Abgabe an unterschiedlichen Positionen des Körpergewebes verbindbar sind.

## Beschreibung

Die Erfindung betrifft eine Hochfrequenz-Applikationsvorrichtung zum vaskulären Einsatz, insbesondere zur Applikation von Hochfrequenz (HF)-Energie an der Renal-Arterienwand.

Durch offenkundige Vorbenutzung ist eine solche Hochfrequenz-Applikationsvorrichtung bekannt, die einen Katheter mit einem ihn in Längsrichtung durchsetzenden Lumen, einem im Lumen geführten, selbstexpandierenden, stentartigen Träger und einen am Träger angeordneten HF-Applikator zur Abgabe von HF-Energie an Körpergewebe aufweist.

Diese bekannte HF-Applikationsvorrichtung weist nur einen einzigen HF-Applikator als Einzelpol-Ablationselektrode auf. Sollen in einem Körperhohlraum oder Körpergefäß an mehreren zueinander versetzten Positionen HF-Energien zu therapeutischen Zwecken auf-gebracht werden, wie dies beispielsweise bei der RSD-(= Renal Sympathetic Denervation-)Therapie angewendet wird, so ist mit dieser einzigen Ablationselektrode der Nachteil verbunden, dass die Prozedur mehrere Male je Gefäß an verschiedenen Positionen wiederholt werden muss, um einen Erfolg der Therapie zu gewährleisten. Bis zu sechs Wiederholungen pro Gefäß sind üblich. Da jeder einzelne Ablationspunkt bis zu zwei Minuten mit HF-Energie beaufschlagt werden muss, ist der gesamte Eingriff sehr zeitintensiv.

Die Einzelpol-Methode lässt darüber hinaus keine sehr präzise Positionierung der Ablationspunkte zu, da nach jedem HF-Energieeintrag der Katheter sowohl axial als auch in Umfangsrichtung um eine gewisse Strecke per Hand verschoben werden muss.

Andere, durch offenkundige Vorbenutzung bekannte Ansätze zur Lösung der vorstehenden Problematik basieren auf der Verwendung eines Ballonkatheters. Dies hat allerdings den Nachteil, dass eine gekrümmte Arterie bei der Ballon-Dilatation gerade gerichtet wird, womit die Gefahr einer Ruptur des Gefäßes verbunden ist. Zudem müssen für unterschiedliche Gefäßdurchmesser unterschiedlich große Ballone eingesetzt werden.

Schließlich haben geftochtene Stent-Designs den Nachteil, eine sehr hohe Längenänderung bei ihrer Expansion zu zeigen. Dies erschwert eine exakte Positionierung der Ablationspunkte ebenfalls. Zudem lässt sich die Anpresskraft der Elektroden an der Arterienwand nur schwierig einstellen.

Ausgehend von den geschilderten Nachteilen des Standes der Technik liegt der Erfindung die Aufgabe zugrunde, eine Hochfrequenz-Applikationsvorrichtung zum vaskulären Einsatz zu schaffen, bei der eine Abgabe von HF-Energie an mehreren Stellen gleichzeitig erfolgen kann.

Diese Aufgabe wird laut Kennzeichnungsteil des Anspruches 1 durch eine Hochfrequenz-Applikationsvorrichtung gelöst, bei der der HF-Applikator als Multipol-Anordnung eine Mehrzahl von über dem Träger axial und peripher verteilt angeordneten HF-Kontaktelementen aufweist. Diese sind vom Träger isoliert und mit einer HF-Quelle zur simultanen oder sequenziellen HF-Energie-Abgabe an unterschiedlichen Positionen des Körpergewebes verbindbar.

Die erfindungsgemäße HF-Applikationsvorrichtung bietet also die Möglichkeit, HF-Energie an mehreren Positionen gleichzeitig beispielsweise an die innere Gefäßwand einer Renalarterie abzugeben. Damit erfolgt der Ablationsprozess je Arterie nur über eine kurze Zeitdauer, wie sie beim Stand der Technik für die Beaufschlagung eines einzigen Ablationspunktes benötigt werden würde. Die Dauer der schmerzhaften Ablationsprozedur wird damit um ein Vielfaches verkürzt. Die HF-Kontaktelemente können aufgrund der Anordnung auf einem selbstexpandierenden, stentartigen Träger (beispielsweise aus Shape-Memory-Metall wie Nitinol) in der Arterie freigesetzt werden. Nach Abgabe der HF-Energie wird der Träger wieder in den Katheter zurückgezogen bzw. der Katheter wieder über den Träger geschoben und kann somit aus der Arterie entfernt werden. Das System ist also nicht nur selbstexpandierend, sondern auch reponierbar.

In den abhängigen Ansprüchen sind bevorzugte Weiterbildungen der erfindungsgemäßen Hochfrequenz-Applikationsvorrichtung charakterisiert. So können die HF-Kontaktelemente jeweils eine freigestellte Kontaktzone und mindestens einen Verbindungssteg zu ihrer mechanischen Anbindung an den Träger aufweisen. Durch diese Ausbildung sind die einzelnen Funktionen der mechanischen Halterung der Kontaktelemente und der HF-Energieabgabe unterschiedlichen Bauteilen, nämlich der Kontaktzone und dem Verbindungssteg, zugewiesen. Damit können diese Bauteile jeweils optimal auf ihre Aufgabe zugeschnitten werden.

Ferner kann die Hochfrequenz-Applikationsvorrichtung im Bereich der Kontaktzonen jeweils so ausgebildet sein, dass diese zwischen einer am nicht-expandierten Träger anliegenden oder darin eingebetteten Passivstellung und einer über die Außenkontur des expandierten Trägers radial abstehenden Aktivstellung lagevariabel sind. Damit ragen die therapeutisch wirksamen Kontaktzonen radial über die Randkontur der stentähnlichen Trägerstruktur hinaus und stellen auch in gewundenen Passagen von Arterien einen ausreichenden Kontakt zur Gefäßwand sicher. Die Kontaktzonen sind dabei in definierten axialen und peripheren Abständen zueinander angeordnet, wodurch ein Therapieerfolg in zuverlässig vorhersehbarer Weise zu erzielen ist.

Für die Kontaktzonen sind unterschiedliche Ausgestaltungen denkbar. So kann die Kontaktzone als geschlossene therapeutische Kontaktfläche mit flacher, kellenartiger Form ausgebildet sein. Diese Kontaktzonen sind in herstellungstechnisch relativ einfacher Weise zusammen mit der Trägerstruktur beispielsweise durch Herausschneiden aus einem Rohrmaterial zu fertigen.

Alternativ dazu kann die Kontaktzone einen mechanischen Halter ausbilden, an dem eine separate therapeutische Kontaktfläche angeordnet ist. Diese kann beispielsweise als HF-Elektrodenkopf gestaltet sein, der in einer Aufnahme des Halters angeordnet ist.

In vorteilhafter Weise ist der HF-Elektrodenkopf dann gegenüber dem Halter durch eine lokale Isolierung galvanisch entkoppelt und idealer weise gleichzeitig thermisch möglichst gut an die metallische Trägerstruktur angekoppelt. Dazu kann die HF-Elektrode beispielsweise mit thermisch leitfähigen aber elektrisch isolierenden Klebstoffen an oder in die metallische Tragstrukur geklebt werden. Ebenso ist es möglich, die HF-Elektrode mit einem Polymer in die metallische Tragstruktur einzugießen.

Die Energieversorgung des HF-Elektrodenkopfes kann in üblicher Weise über einzelne Drähte erfolgen, vorteilhaft ist allerdings eine Energieversorgung über eine am Träger sitzende Platine, auf der der HF-Elektrodenkopf montiert ist.

Zur galvanischen Entkopplung können auch einzelne Ringflächen zur Ausbildung der therapeutischen Kontaktflächen der jeweiligen Kontaktzonen am Träger vorgesehen sein.

Eine weitere Alternative für die Isolierung der Kontaktzone besteht in einer Isolationsschicht, beispielsweise einer dünnen Kunststoffschicht, als Überzug auf dem gesamten oder einem Teil des Trägers.

Durch eine weiterhin mögliche längenflexible Ausbildung der Verbindungsstege der Kontaktelemente zwischen der jeweiligen Kontaktzone und dem Träger, welche insbesondere mäanderförmig verlaufen, kann eine zuverlässige Positionierung der Kontaktzonen in variabler Weise relativ zum Träger erzielt werden. Dadurch kann eine sichere Kontaktierung der Applikationsvorrichtung an der Gefäßwand unterstützt werden.

Da bei HF-Applikationen in Gefäßen eine punktgenaue Temperaturüberwachung der energiebeaufschlagten Stelle von Vorteil ist, können in oder an den HF-Kontaktelementen ein oder mehrere Temperatursensoren vorgesehen sein. Damit ist eine permanente Temperaturkontrolle in der Nähe der therapeutischen Kontaktfläche möglich.

Weitere bevorzugte Ausführungsformen kennzeichnen an sich bekannte Grundstrukturen für den stentartigen, selbstexpandierenden Träger. Dieser kann nach Art eines so genannten "Slotted Tube Stents" - im Folgenden auch kurz "Slotted Tube" genannt - ausgeführt sein. Diese Stentstruktur wird beispielsweise mittels eines Laserstrahls aus einem Rohr geschnitten und bildet deswegen im Gegensatz zu einer Flechtstruktur ("Braided Design") eine in sich geschlossene Struktur. Zudem weist diese Stentstruktur keine praktisch relevante Längenänderung bei der Expansion auf, wodurch eine sehr genaue Positionierung der Ablationspunkte in Umfangs- und Axialrichtung möglich ist. Damit werden signifikante Vorteile gegenüber dem aus dem Stand der Technik bekannten Einzelpol-Ablationsgerät erzielt, das auf einer Flechtstruktur ("Braided Design") des Stents beruht. Die Erfolgsquote bei der Verödung der sympathetischen Nerven etwa in der Renalarterie steigt gegenüber diesen Braided Design Stents sowie einer Einzelpolanwendung signifikant.

Weitere Vorteile der "Slotted Tube Stents" gegenüber dem "Braided Design" liegt in der kleineren Profilausdehnung, da die Kreuzungspunkte des beim Braided Design vorhandenen Drahtgeflechts entfallen. Ferner ist eine leichtere Isolationsmöglichkeit durch eine Polymerbeschichtung nach dem Einprägen der Form realisierbar. Isolierte Drähte beim Braided Design ermöglichen keine Temperaturbehandlung zur Formeinprägung in der Stentstruktur. Weiterhin weisen Slotted Tube Stents als Trägerstruktur eine geringere Torsionssteifigkeit als extrem torsionssteife Braided Design Strukturen auf. Auch ist die Einstellung der Radialkraft der HF-Kontaktelemente, die beispielsweise als kellenartige Ansätze ausgebildet sind, besser realisierbar.

Gegenüber der Ballontechnologie ist als Vorteil eine größere Flexibilität des Stentdesigns zu nennen. Gekrümmte Gefäße werden während der Behandlung (dilatierter Ballon) nicht begradigt, wodurch die Gefahr von Gefäßverletzungen verringert wird. Darüber hinaus wird der Blutfluss durch die Netzmaschen des "Slotted Tube Stents" praktisch nicht unterbrochen.

Eine Alternative für die Ausbildung des Trägers ist eine Art Stentgraft oder die Kombination aus mehreren selbstexpandierenden Ringsegmenten, die auf einem im Katheter verschiebbaren Lagerschaft aneinandergereiht montiert sind. Beide Versionen zeigen ähnliche Vorteile wie die oben erörterten "Slotted Tube Stents".

Gemäß einer weiteren Forbildung aller Ausführungen, Varianten und Alternativen der beschriebenen Hochfrequenz-Applikationsvorrichtung sind die HF-Kontaktelemente aus einem Material mit einem guten Röntgenkontrast gefertigt.

Weitere Merkmale, Einzelheiten und Vorteile der Erfindung ergeben sich aus der nachfolgenden Beschreibung von Ausführungsbeispielen anhand der Zeichnungen. Es zeigen:
- Fig. 1: eine schematische Gesamtansicht einer HochfrequenzApplikationsvorrichtung,
- Fig. 2: eine schematische Draufsicht in Abwicklung eines HF-Applikators mit einer "Slotted Tube"-Struktur,
- Fig. 3: das distale Ende der HF-Applikationsvorrichtung gemäß Fig. 1 mit HF-Applikator in Aktivstellung,
- Fig. 4: eine schematische Draufsicht in Abwicklung eines HF-Applikators mit einer Stentgraft-Struktur,
- Fig. 5: eine schematische Ansicht des distalen Endes einer HochfrequenzApplikationsvorrichtung mit einem HF-Applikator aus mehreren selbstexpandierenden Ringsegmenten,
- Fig. 6: eine Draufsicht auf ein HF-Kontaktelement in einer ersten Ausführungsform,
- Fig. 7: eine Draufsicht auf ein HF-Kontaktelement in einer zweiten Ausführungsform,
- Fig. 8: einen Axialschnitt des HF-Kontaktelements entlang der Schnittlinie A-A gemäß Fig. 6,
- Fig. 9 und 10: einen Axialschnitt des HF-Kontaktelements analog Fig. 8 in zwei weiteren unterschiedlichen Ausführungsformen,
- Fig. 11 und 12: Draufsichten in Abwicklung auf einen HF-Applikator mit einem Träger mit "Slotted Tube"-Struktur in kollabiertem und expandiertem Zustand,
- Fig. 13: eine schematische perspektivische Ansicht des HF-Applikators gemäß Fig. 11 und 12 in expandiertem Zustand,
- Fig. 14 und 15: Draufsichten in Abwicklung auf einen HF-Applikator in einer weiteren Ausführungsform in kollabiertem und expandiertem Zustand, sowie
- Fig. 16: eine Draufsicht in Abwicklung auf einen als Einzelsegment ausgebildeten HF-Applikator.

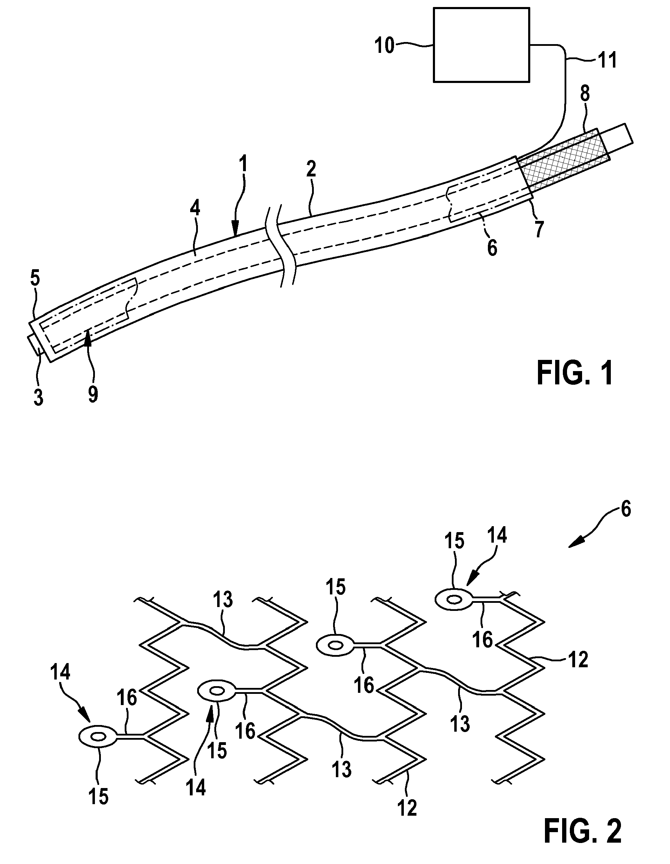

Wie aus Fig. 1 deutlich wird, weist eine Hochfrequenz-Applikationsvorrichtung zum vaskulären Einsatz einen als langgestreckten Schlauch ausgebildeten Katheter 1 mit einem Außenschaft 2 und einem darin angeordneten Innenschaft 3 auf. Zwischen diesen ist ein ringförmiges Lumen 4 gebildet, das den Katheter 1 in Längsrichtung durchsetzt.

In diesem Lumen 4 ist ein zumindest am distalen Ende 5 stentartiger Träger 6 angeordnet, der an seinem proximalen Ende 7 durch eine schematisch angedeutete Betätigungsmimik 8 in noch näher zu beschreibender Weise zu betätigen ist. Am distalen Ende 5 des Trägers 6 ist ein als Ganzes mit 9 bezeichneter HF-Applikator vorgesehen, um HF-Energie beispielsweise zur ganz oder teilweisen Durchtrennung oder Traumatisierung sympathetischer Nerven an der Renalarterie zur dauerhaften Therapie chronischen Bluthochdrucks anzulegen. Diese HF-Energie dient in der Regel nicht dazu, den Nerv physiologisch komplett zu durchtrennen oder zu zerstören, sondern um ihn durch die HF-Energie induzierte Prozesse infunktionell zu machen.

In Fig. 1 ist rein schematisch eine HF-Quelle 10 für die Energieversorgung des HF-Applikators 9 dargestellt, die über eine geeignete Leitung 11 mit dem HF-Applikator 9 verbunden ist.

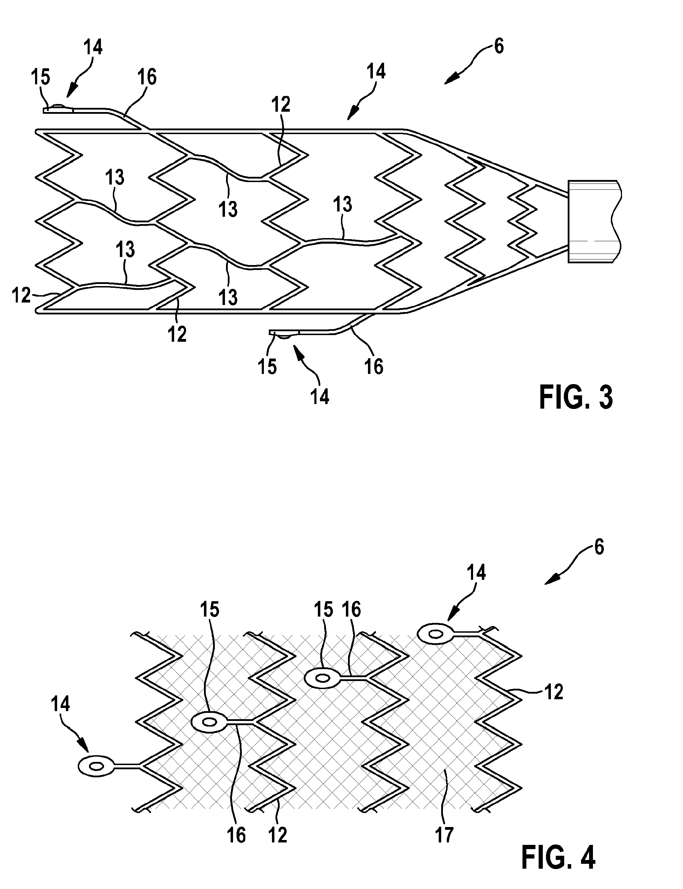

Eine erste Ausführungsform für den HF-Applikator 9 ist in Fig. 2 und 3 dargestellt. Der Träger 6 ist hier nach Art eines "Slotted Tube Stents" ausgebildet, der aus Haupt-Mäanderstreben 12 und longitudinalen Brückenstreben 13 einen Art Netzstruktur bildet. An verschiedenen Mäanderpunkten der Haupt-Mäanderstreben 12 sind über den Träger 6 HF-Kontaktelemente 14 verteilt, die jeweils als Elektrode mit der HF-Quelle 10 zur HF-Energie-Abgabe an unterschiedlichen Positionen des Körpergewebes verbindbar sind.

Bei der Anwendung der Hochfrequenz-Applikationsvorrichtung wird der Katheter 1 mit in sein Lumen 4 zurückgezogenem Träger 6 - wie dies in Fig. 1 angedeutet ist - an die entsprechende Körperposition mit seinem distalen Ende 5 vorgeschoben. Dort wird der Außenschaft des Katheters 1 zurückgezogen, so dass sich der selbstexpandierende Träger 6 beim Austreten aus dem Lumen 4 aufweitet, wie dies in Fig. 3 gezeigt ist.

Die HF-Kontaktelemente 14 sind dabei jeweils durch eine vom umgebenden Material des Trägers 6 durch entsprechendes Ausschneiden freigestellte Kontaktzone 15 um einen diese tragenden Verbindungssteg 16 zu ihrer mechanischen Anbindung an den Träger 6 gebildet. Wie aus Fig. 3 deutlich wird, verlagern sich durch das Expandieren des Trägers 6 die Kontaktzonen 15 der HF-Kontaktelemente 14 radial nach außen, so dass eine zuverlässige Anlage der Kontaktzonen 15 an das den HF-Applikator 9 umgebende Körpergewebe beispielsweise der Renalarterie gewährleistet ist. In diesem Zustand können dann die Kontaktzonen 15 von der HF-Quelle 10 mit entsprechender HF-Energie versorgt und an den Kontaktpunkten entsprechende Ablationen zu therapeutischen Zwecken vorgenommen werden.

In Fig. 4 ist eine alternative Ausführungsform für den Träger 6 dargestellt, der hier nach Art eines Stentgrafts ausgebildet ist. Dieser weist wiederum Haupt-Mäanderstreben 12 auf, die allerdings durch ein flexibles Gewebe 17 in longitudinaler Richtung miteinander verbunden sind. Analog der Ausführungsform gemäß Fig. 2 und 3 sitzen an den Haupt-Mäanderstreben 12 wiederum HF-Kontaktelemente 14.

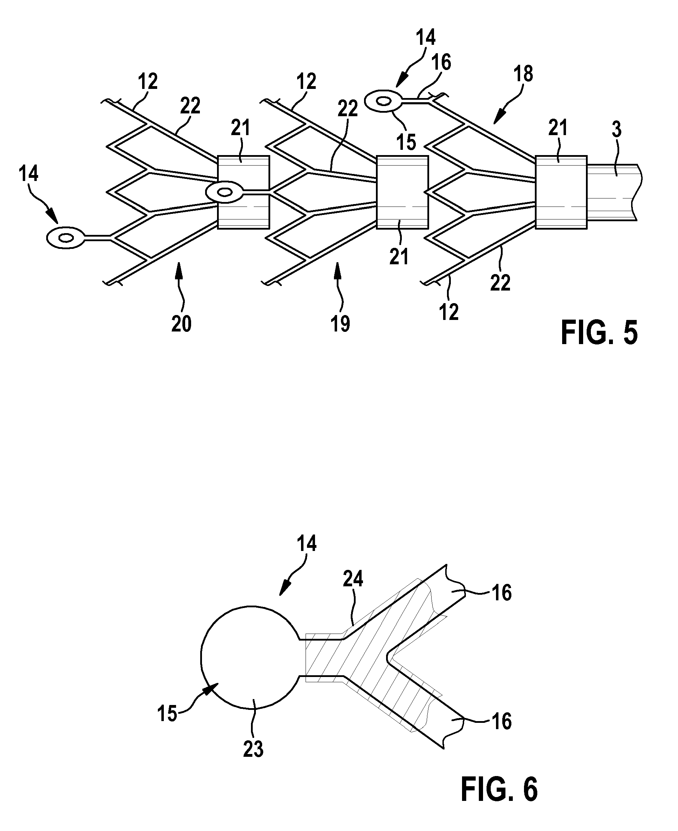

Bei der in Fig. 5 gezeigten Ausführungsform ist der Träger 6 aus mehreren selbstexpandierenden Ringsegmenten 18, 19, 20 gebildet, die über Hülsen 21 jeweils auf dem Innenschaft 3 des Katheters 1 befestigt sind. Jedes Ringsegment zeigt analog den Ausführungsformen gemäß Fig. 2 und 4 wieder Haupt-Mäanderstreben 12 mit daran angesetzten HF-Kontaktelementen 14. Die Haupt-Mäanderstreben 12 sind dabei über longitudinale Koppelstreben 22 an die Hülsen 21 angebunden. Wie anhand von Fig. 5 gut nachvollziehbar ist, werden die Ringsegmente 18, 19, 20 beim Zurückziehen des Innenschafts 3 in den Außenschaft 2 des Katheters 1 schirmartig zusammengefaltet, wodurch sich die stentartige Ringsegment-Struktur kontrahiert. Umgekehrt expandieren die Ringsegmente 18, 19, 20 beim Zurückziehen des Außenschafts 2 über den Innenschaft 3, wodurch sich die HF-Kontaktelemente 14 wiederum an die Gefäßinnenwand anlegen.

Anhand der Fig. 6 bis 10 sind unterschiedliche Ausführungsformen der HF-Kontaktelemente 14 zu erläutern. So zeigt Fig. 6 ein HF-Kontaktelement 14, dessen Kontaktzone 15 als geschlossene therapeutische Kontaktfläche 23 mit flacher, rundkellenartiger Form ausgebildet ist. Diese ist in geeigneter Weise, wie beispielsweise durch eine dünne Kunststoffbeschichtung 24 galvanisch von den Verbindungsstegen 16 und damit dem restlichen Träger 6 entkoppelt.

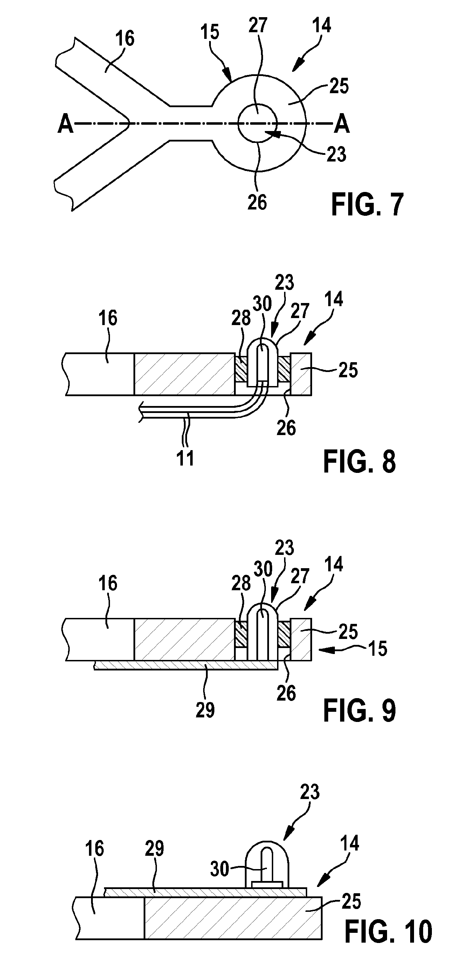

Die in Fig. 7 und 8 dargestellte Variante zeigt ein HF-Kontaktelement 14 mit einer Kontaktzone 15, die einen ringförmigen mechanischen Halter 25 in Form eines Durchbruchs 26 ausbildet. In diesem Durchbruch 26 ist ein HF-Elektrodenkopf 27 als therapeutische Kontaktfläche 23 untergebracht, der über einen geeigneten Ringisolator 28 im Durchbruch 26 galvanisch isoliert ist. Der Elektrodenkopf 27 selbst wird - wie Fig. 8 ebenfalls zeigt - über die oben erwähnten Leitungen 11 mit HF-Energie versorgt.

Bei der in Fig. 9 dargestellten Ausführungsform sitzt der HF-Elektrodenkopf 27 ebenfalls über den Ringisolator 27 galvanisch entkoppelt in dem Durchbruch 26 des mechanischen Halters 25, der von der Kontaktzone 15 ausgebildet ist, jedoch ist hier eine Platine 29 unterhalb der Kontaktzone 15 vorgesehen, auf der der HF-Elektrodenkopf 27 montiert und entsprechend über nicht näher dargestellte Leiterbahnen an die HF-Quelle 10 angeschlossen ist.

Bei der Ausführungsform gemäß Fig. 10 ist der HF-Elektrodenkopf 27 ebenfalls auf einer Platine 29 montiert, wobei diese jedoch auf dem mechanischen Halter 25 sitzt, so dass der Durchbruch 26 entfallen kann. Die Energieversorgung des HF-Elektrodenkopfes 27 erfolgt wiederum über Leiterbahnen auf der Platine 29.

Bei den in Fig. 7 bis 10 gezeigten HF-Elektrodenköpfen 27 ist ein Temperatursensor 30 integriert, der einer Temperaturerfassung in unmittelbarer Nähe der Ablationsstelle dient. Damit ist eine besonders zuverlässige Steuerung der Beaufschlagung des Körpergewebes mit HF-Energie gewährleistet.

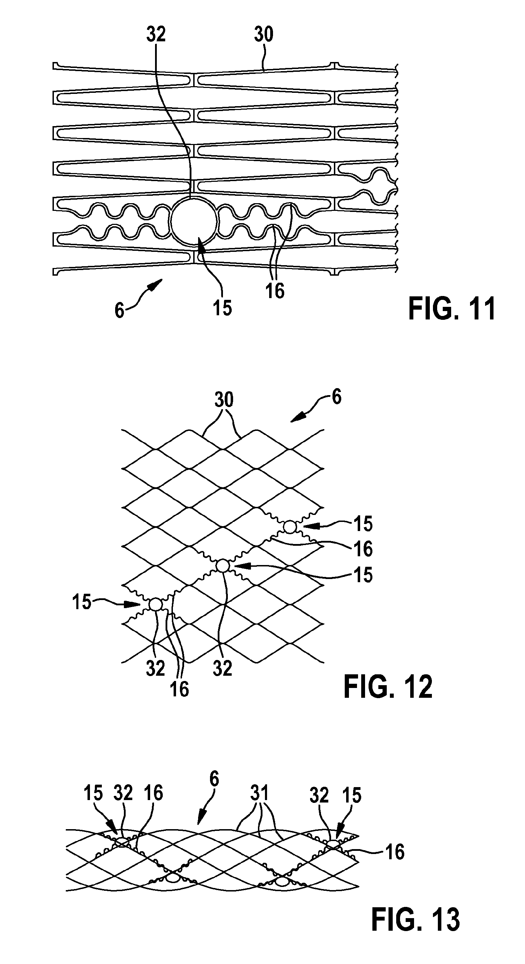

Die Fig. 11, 12 und 13 zeigen einen Träger 6 auf der Basis einer "Slotted Tube"-Struktur mit rautenförmig angeordneten Gitterstreben 31, wobei an verschiedenen Stellen dieser Struktur als Kontaktzonen 15 Ringflächen 32 ausgebildet sind, die über mäanderförmige Verbindungsstege 16 an die Struktur des Trägers 6 angebunden sind.

Wie aus Fig. 12 und 13 hervorgeht, gleichen die mäanderförmigen Verbindungsstege 16 die Expansionsbewegung der Gitterstreben 31 aus und sorgen dafür, dass die Ringflächen 32 radial weit außen bleiben und über die Kontur des Trägers 6 radial hinausstehen.

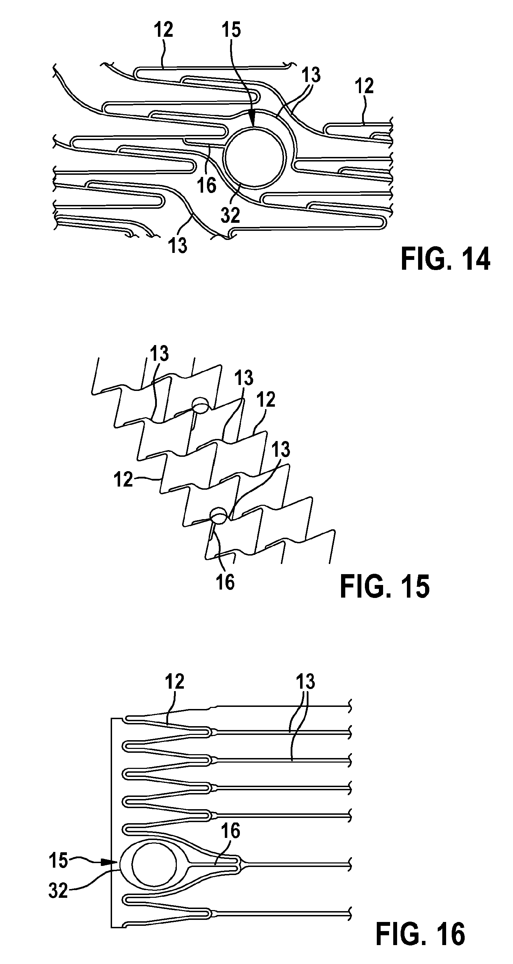

In den Fig. 14 und 15 ist ein weiteres Beispiel für ein Trägerdesign mit Haupt-Mäanderstreben 12, geschwungenen longitudinalen Brückenstreben 13 und einer als Ringfläche 32 ausgeführten Kontaktzone 15 der HF-Kontaktelemente 14 gezeigt. Die Kontaktzonen 15 sind dabei über einen einzigen, schmalen Verbindungssteg 16 an die Haupt-Mäanderstreben 12 angebunden. Wie aus Fig. 15 hervorgeht, gleiten die Ringflächen 32, die in der kontrahierten Stellung in die Struktur zwischen zwei bogenförmigen Brückenstreben 13 eingebettet sind, beim Expandieren über die Brückenstreben 13 nach außen, wodurch der Kontakt mit dem umliegenden Gewebe wiederum gewährleistet ist.

Die in den Fig. 11 bis 13 bzw. 14 und 15 gezeigten Grund-Strukturen des Trägers 6 sind - eben bis auf die erfindungsgemäßen Ergänzungen - als sogenanntes geschlossen-zelliges "Slotted Tube"-Design ("Closed cell design") grundsätzlich bekannt.

In Fig. 16 schließlich ist ein Einzelsegment mit Haupt-Mäanderstreben 12 und longitudinal verlaufenden Brückenstreben 13 dargestellt, wobei zwischen zwei Mäanderbögen eine als Ringfläche 32 ausgebildete Kontaktzone 15 wiederum über einen Verbindungssteg 16 an die Haupt-Mäanderstreben 12 angebunden ist.

## Patentansprüche

1. Hochfrequenz-Applikationsvorrichtung zum vaskulären Einsatz, insbesondere zur Applikation von Hochfrequenz(HF)-Energie an der Renal-Arterienwand, umfassend
- einen Katheter (1) mit einem ihn in Längsrichtung durchsetzenden Lumen (4),
- einen im Lumen (4) geführten, selbstexpandierenden, stentartigen Träger (6), und
- einen am Träger (6) angeordneten HF-Applikator (9) zur Abgabe von HF-Energie an Körpergewebe,
**dadurch gekennzeichnet, dass**
- der HF-Applikator (9) als Multipol-Anordnung eine Mehrzahl von über den Träger (6) axial und peripher verteilt angeordneten HF-Kontaktelementen (14) aufweist, die vom Träger (6) isoliert sind und mit einer HF-Quelle (10) zur simultanen oder sequenziellen HF-Energie-Abgabe an unterschiedlichen Positionen des Körpergewebes verbindbar sind.

2. Hochfrequenz-Applikationsvorrichtung nach Anspruch 1, **dadurch gekennzeichnet, dass** die HF-Kontaktelemente (14) jeweils eine freigestellte Kontaktzone (15) und mindestens einen Verbindungssteg (16) zu ihrer mechanischen Anbindung an den Träger (6) aufweisen.

3. Hochfrequenz-Applikationsvorrichtung nach Anspruch 2, **dadurch gekennzeichnet, dass** die Kontaktzone (15) des jeweiligen HF-Kontaktelementes (14) zwischen einer am nicht-expandierten Träger (6) anliegenden oder darin eingebetteten Passivstellung und einer über die Außenkontur des expandierten Trägers (6) radial abstehenden Aktivstellung lagevariabel ist.

4. Hochfrequenz-Applikationsvorrichtung nach Anspruch 2 oder 3, **dadurch gekennzeichnet, dass** die Kontaktzone (15) als geschlossene therapeutische Kontaktfläche (23) mit flacher, kellenartiger Form ausgebildet ist.

5. Hochfrequenz-Applikationsvorrichtung nach Anspruch 2 oder 3, **dadurch gekennzeichnet, dass** die Kontaktzone (15) einen mechanischen Halter (25) bildet, an dem eine therapeutische Kontaktfläche (23) angeordnet ist.

6. Hochfrequenz-Applikationsvorrichtung nach Anspruch 5, **dadurch gekennzeichnet, dass** die therapeutische Kontaktfläche (23) als HF-Elektrodenkopf (27) ausgebildet ist, der in einer Aufnahme (26) des Halters (25) angeordnet ist.

7. Hochfrequenz-Applikationsvorrichtung nach Anspruch 6, **dadurch gekennzeichnet, dass** der HF-Elektrodenkopf (27) gegenüber dem Halter (25) durch eine lokale Isolierung (28) galvanisch entkoppelt ist.

8. Hochfrequenz-Applikationsvorrichtung nach Anspruch 6 oder 7, **dadurch gekennzeichnet, dass** der HF-Elektrodenkopf (27) auf einer am Halter (25) sitzenden Platine (29) zur Energieversorgung montiert ist.

9. Hochfrequenz-Applikationsvorrichtung nach mindestens Anspruch 2 oder 3, **dadurch gekennzeichnet, dass** die therapeutische Kontaktfläche (23) der Kontaktzone (15) als Ringfläche (32) ausgebildet ist.

10. Hochfrequenz-Applikationsvorrichtung nach einem der vorgenannten Ansprüche, **dadurch gekennzeichnet, dass** die Isolierung der Kontaktzonen (15) durch eine Isolationsschicht (24) auf dem Träger (6) erfolgt.

11. Hochfrequenz-Applikationsvorrichtung mindestens nach Anspruch 2, **dadurch gekennzeichnet, dass** der Verbindungssteg (16) längenflexibel, insbesondere durch einen mäanderförmigen Verlauf, ist.

12. Hochfrequenz-Applikationsvorrichtung nach einem der vorgenannten Ansprüche, **dadurch gekennzeichnet, dass** in oder an den HF-Kontaktelementen (14) ein oder mehrere Temperatursensoren (30) angeordnet sind.

13. Hochfrequenz-Applikationsvorrichtung nach einem der vorgenannten Ansprüche, **dadurch gekennzeichnet, dass** der Träger (6) nach Art eines Slotted Tube Stents ausgebildet ist.

14. Hochfrequenz-Applikationsvorrichtung nach einem der Ansprüche 1 bis 11, **dadurch gekennzeichnet, dass** der Träger (6) nach Art eines Stentgrafts ausgebildet ist.

15. Hochfrequenz-Applikationsvorrichtung nach einem der Ansprüche 1 bis 12, **dadurch gekennzeichnet, dass** der Träger (6) aus mehreren selbstexpandierenden Ringsegmenten (18, 19, 20) zusammengesetzt ist, die auf einem im Katheter (1) verschiebbaren Lagerschaft (3) aneinandergereiht montiert sind.

16. Hochfrequenz-Applikationsvorrichtung nach einem der Ansprüche 1 bis 15, **dadurch gekennzeichnet, dass** die HF-Kontaktelemente aus einem Material mit einem guten Röntgenkontrast gefertigt sind.
